# EUROPÄISCHE PATENTSCHRIFT

(11) **EP 2 064 309 B1**
(45) Veröffentlichungstag und Bekanntmachung des Hinweises auf die Patenterteilung: **04.05.2016**
(21) Anmeldenummer: 07800168.2
(22) Anmeldetag: 06.09.2007
(51) Int. Cl.: C12M 1/107, C12M 1/08

(54) **BIOGASANLAGE**
BIOGAS SYSTEM
STATION DE GAZ BIOLOGIQUE

(30) Priorität: 21.09.2006 AT 15782006
(43) Veröffentlichungstag der Anmeldung: 03.06.2009
(73) Patentinhaber: Universität Innsbruck, 6020 Innsbruck (AT)
(72) Erfinder: WETT, Bernhard, A-6020 Innsbruck (AT)
(74) Vertreter: Schwarz & Partner
(86) Internationale Anmeldenummer: PCT/AT2007/000423
(87) Internationale Veröffentlichungsnummer: WO 2008/034153

(56) Entgegenhaltungen:
- EP-A- 0 172 443
- CH-A5- 688 737
- DE-A1- 3 604 415

## Beschreibung

Die vorliegende Erfindung bezieht sich auf eine Biogasanlage mit einem Fermenter, der eine erste Gärkammer und zumindest eine zweite Gärkammer zum Vergären des Gärmediums aufweist, wobei in der ersten Gärkammer gebildetes Biogas in ein in der zweiten Gärkammer angeordnetes Steigrohr einbringbar ist.

In Gärkammern wird aus organischen Substanzen, wie z.B. Klärschlamm, Gülle, Lebensmittelabfällen, Pflanzenschnitt und anderen landwirtschaftlichen Reststoffen energiereiches Biogas gewonnen. Dieses Biogas kann in Maschinen, wie Gasmotoren und Turbinen, in Wärme und in elektrische Energie umgewandelt werden. Den Erzeugern von biogenen Gasen wurde im Rahmen der Liberalisierung des österreichischen Gasmarktes zudem die Möglichkeit eröffnet, in das öffentliche Erdgasnetz einzuspeisen, sofern die festgelegten Qualitätsanforderungen erfüllt werden. Voraussetzung für eine rasche Fermentierung und eine effektive Biogasproduktion ist eine homogene Durchmischung des Gärmediums in den Gärkammern, sodass sich die im Gärmedium befindlichen Feststoffe nicht am Boden ablagern, sondern in Schwebe gehalten werden. Den Stand der Technik der Rührsysteme für Biogasanlagen repräsentieren unter anderem mechanische Rührsysteme, z.B. langsam drehende Paddelrührwerke mit Vertikalachse oder schnell drehende Propellerrührer. Diese Rührsysteme weisen neben den hohen Anschaffungskosten auch den Nachteil eines intensiven Wartungserfordernisses auf.

CH 688 737 A5 offenbart eine Biogasanlage mit einem Fermenter, der mehrere Gärkammern zum Vergären eines Gärmediums aufweist, wobei gebildetes Biogas an der letzten Gärkammer entnommen und in ein Steigrohr der letzten Gärkammer eingebracht wird. EP 0 172 443 A1 offenbart eine Biogasanlage mit einem Fermenter, der drei Gärkammern zum Vergären des Gärmediums aufweist, wobei in der zweiten Gärkammer ein Steigrohr angeordnet ist.

Aufgabe der vorliegenden Erfindung ist es daher, eine Biogasanlage der eingangs erwähnten Gattung mit homogener Durchmischung des Gärmediums vorzuschlagen, wobei der Einsatz von mechanischen Rührwerken nicht zwingend erforderlich ist.

Dies wird erfingdungsgemäß dadurch erreicht, dass eine Gasleitung von der ersten Gärkammer zur zweiten Gärkammer führt und an einer Eintrittsstelle in das in der zweiten Gärkammer angeordnete Steigrohr mündet, sodass in der ersten Gärkammer gebildetes Biogas in das Steigrohr einbringbar ist, wobei das Steigrohr eine Einlassöffnung und eine Auslassöffnung zum Durchtritt des Gärmediums aufweist.

Auf diese Weise wird eine Einrichtung für den weitgehenden anaeroben Abbau organischer Substrate unter Nutzung des Gasdrucks des in der ersten Gärkammer produzierten Biogases geschaffen, das in das Steigrohr der zweiten Gärkammer einpressbar ist. Das - vorzugsweise über wenigstens einen Großteil der maximalen Höhe der Gärkammer erstreckende - Steigrohr der zweiten Gärkammer basiert auf dem Grundprinzip einer Mammutpumpe, bei dem durch den Eintrag des Biogases ein Gärmedium-Biogas-Gemisch mit einem wesentlich niedrigeren spezifischen Gewicht als jenes des das Steigrohr umgebenden Gärmediums herbeigeführt wird. Mit anderen Worten verringern die aufsteigenden Gasblasen im Steigrohr der zweiten Gärkammer die Dichte der Flüssigkeit gegenüber der umgebenen Flüssigkeit. Der Dichteunterschied induziert eine aufsteigende Strömung im Steigrohr, der somit der Reaktorumwälzung dient.

Es ist vorgesehen, dass das Steigrohr derart ausgebildet ist, dass das Gärmedium an einer Einlassöffnung in das Steigrohr gelangt und an einer Auslassöffnung wieder aus dem Steigrohr austritt. In diesem Zusammenhang ist vorgesehen, dass die Einlassöffnung unterhalb der Auslassöffnung angeordnet ist. Für einen optimalen Vergärungsprozess kann es vorteilhaft sein, wenn das Steigrohr in der Gärkammer im Wesentlichen vertikal und vorzugsweise mittig angeordnet ist.

Um zu erreichen, dass schon ein relativ geringer Gasdruck des produzierten Biogases ausreicht, um den hydrostatischen Druck der Flüssigkeitssäule zu überwinden und damit eine aufsteigende Strömung im Steigrohr zu induzieren, kann es vorteilhaft sein, wenn das Biogas im unteren Bereich, vorzugsweise in der untersten Hälfte, des obersten Drittels des Steigrohres einbringbar ist.

Gemäß einer bevorzugten Ausgestaltung der Erfindung kann vorgesehen sein, dass das Steigrohr eine Heizeinrichtung, vorzugsweise einen Wärmetauscher, aufweist. Auf diese Weise kann der Reaktor beheizt werden, wobei durch die erhöhte Temperatur der Flüssigkeit im Steigrohr ein zusätzlicher Dichteunterschied gegenüber der Flüssigkeit im umgebenden Reaktorraum generiert wird. In diesem Zusammenhang kann es von Vorteil sein, wenn das Steigrohr zumindest bereichsweise doppelwandig ausgeführt ist, wobei zwischen den beiden Wandungen ein Heizfluid, vorzugsweise Heizwasser, zirkulierbar ist.

Das Heizwasser kann beispielsweise von der Überwärme eines Blockheizkraftwerkes dem Heberrohr zugeführt werden. Durch die dadurch verbesserte Rührströmung kann außerdem ein guter Wärmeübertrag erzielt werden.

Gemäß einem Ausführungsbeispiel der Erfindung kann vorgesehen sein, dass die Überführung des Biogases von der ersten Gärkammer in das Steigrohr der zweiten Gärkammer über eine, vorzugsweise bis auf einen Einlass und einen Auslass geschlossene, Gasleitung erfolgt. Selbstverständlich können - falls erforderlich - auch Gasventile Verwendung finden, die bei einem vorgegebenen oder vorgebbaren (Über-)Druck in der ersten Gärkammer den Gastransport in das Steigrohr der zweiten Gärkammer erlauben.

Günstigerweise ist vorgesehen, dass die erste Gärkammer wenigstens im befüllten Zustand - bis auf die Gasleitung - gasdicht ausgebildet ist. Auf diese Weise kann der erforderliche Gasdruck im Gasturm der ersten Gärkammer bereitgestellt werden.

Gemäß einem weiteren Ausführungsbeispiel der Erfindung kann vorgesehen sein, dass in der ersten Gärkammer ebenfalls ein Steigrohr angeordnet ist. Dabei kann das Steigrohr alle beschriebenen Merkmale des Steigrohres der zweiten Gärkammer aufweisen. In diesem Zusammenhang kann es von Vorteil sein, wenn in das Steigrohr der ersten Gärkammer Pressluft einbringbar ist, wodurch das Biogas vorteilhaft entschwefelt werden kann.

Weitere Einzelheiten und Vorteile der vorliegenden Erfindung werden anhand der nachstehenden Figurenbeschreibung erläutert. Darin zeigt:
- Fig. 1: eine schematische Schnittdarstellung einer erfindungsgemäßen Biogasanlage in einer Draufsicht,
- Fig. 2: einen Vertikalschnitt der Biogasanlage gemäß Fig. 1, und
- Fig. 3: ein vergrößertes Detail der Gärkammer gemäß Fig. 2.

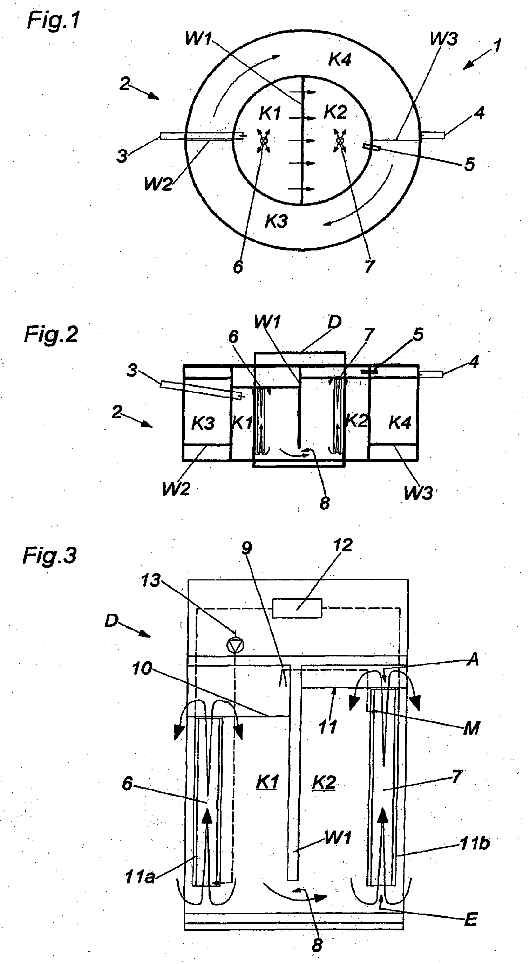

Fig. 1 zeigt eine schematische Draufsicht auf eine erfindungsgemäße Biogasanlage 1 Diese Biogasanlage 1 umfasst einen Fermenter 2, der aus statischen, hydraulischen und wärmetechnischen Gründen eine Rundform aufweist. Der Fermenter 2 beinhaltet eine erste Gärkammer K1 und eine zweite Gärkammer K2, die gemeinsam eine Kreisform beschreiben. Mit dem Bezugszeichen 3 ist ein Einlauf gekennzeichnet, durch den das Gärmedium, wie z.B. Gülle, in die Gärkammer K1 von oben her einbringbar ist. Im Weiteren umfasst der Fermenter 2 zwei Nachgärkammern K3 und K4, wobei eine von der ersten und zweiten Gärkammer gebildete Außenwandung im Wesentlichen konzentrisch zur Außenwandung der beiden Nachgärkammern K3 und K4 angeordnet ist. Die Nachgärkammer K4 weist einen Flüssigkeitsauslass 4 auf. Die Gärkammern K1 und K2 bilden somit den Kern und die Nachgärkammern K3 und K4 die ringförmige Peripherie, wobei die einzelnen Gärkammern K1, K2, K3, K4 durch Wände W1, W2, W3, vorzugsweise Tauchwände, voneinander getrennt sind, sodass im bodennahen Bereich des Fermenters 2 der Durchtritt des Gärmediums von einer Gärkammer in die andere ermöglicht wird, so wie es in Fig. 2 dargestellt ist. Eine klare hydraulische Trennung erfolgt zwischen Kern und Peripherie (beispielsweise zwischen Kammer K2 und K3), wo lediglich in der Höhe des Wasserspiegels eine Überlauföffnung 5 angeordnet ist. Im dargestellten Ausführungsbeispiel ist sowohl in der ersten Gärkammer K1 ein Steigrohr 6 als auch in der zweiten Gärkammer K2 ein Steigrohr 7 angeordnet. Die Erfindung beruht nunmehr auf den Grundgedanken, das durch den Fermentierungsprozess entstehende Biogas in der ersten Gärkammer K1 in ein in der zweiten Gärkammer K2 angeordnetes Steigrohr 7 einzuleiten, um darin eine aufsteigende Strömung zu induzieren. Diese aufsteigende Strömung dient der Reaktorumwälzung, sodass die Feststoffe nicht am Boden des Fermenters 2 abgesetzt werden, sondern in Schwebe gehalten werden und dabei ein homogener Abbau des Substrates erreicht wird. In der ersten Gärkammer K1 kann ebenfalls ein Steigrohr 6 mit den im Rahmen dieser Erfindung beschriebenen Merkmalen angeordnet werden, allerdings wird in das Steigrohr 6 Pressluft anstelle von Biogas in den Thermo-Gas-Lift gedrückt (Luftanteil von ca. 4% hat sich für die Entschwefelung des Biogases bewährt).

Fig. 2 zeigt den Fermenter 2 in einem Vertikalschnitt mit den beiden Gärkammern K1 und K2, die durch eine Tauchwand W1 voneinander getrennt sind, sodass die erste Gärkammer K1 - bis auf eine Gasleitung (Fig. 3), die zum Steigrohr 7 der zweiten Gärkammer K2 führt-oben gasdicht ausgebildet ist und unten den Durchtritt des Gärmediums won einer Gärkammer in die andere (bzw. auch in die umgekehrte Richtung) erlaubt. Die Tauchwand W1 weist hierfür im untersten Bereich, vorzugsweise im untersten Viertel, einen über die Breite der Tauchwand W1 erstreckenden Spalt 8 auf, durch den das Gärmedium hindurchfließen kann. Die Tauchwände W2 und W3 sind in analoger Weise ausgeführt. Mit dem Bezugszeichen D ist ein Detailausschnitt gekennzeichnet, der in Fig. 3 vergrößert dargestellt ist.

Fig. 3 zeigt den vergrößerten Detailausschnitt D aus Fig. 2, anhand dessen das Funktionsprinzip des erfindungsgemäßen Fermenters 2 näher erläutert wird. Das Steigrohr 7 ist derart ausgebildet, dass das Gärmedium an einer Einlassöffnung E in das Steigrohr 7 gelangt und an einer höher gelegenen Austrittstelle A wieder aus dem Steigrohr 7 austritt.

Die Tauchwand W1 dient als Abtrennung der beiden Gärkammern K1 und K2, wobei die Tauchwand W1 im oberen Bereich eine geschlossene Wandung und im unteren Bereich einen Spalt 8 aufweist. Durch die starke Biogasproduktion in der Gärkammer K1 baut sich ein Überdruck im gasdichten Turm der Gärkammer K1 auf und verdrängt den Flüssigkeitsspiegel 10 nach unten und ein entsprechendes Flüssigkeitsvolumen wird unter die tauchwand W1 hindurch in die Gärkammer K2 verdrängt. Der Maximalspiegel 11 in der Gärkammer K2 wird durch die Überlauföffnung 5 (Fig. 1, Fig. 2) zur Nachgärkammer K3 vorgegeben. Eine von der Gärkammer K1 ausgehende Gas-Überdruckleitung 9 wird direkt in das Steigrohr 7 der zweiten Gärkammer K2 an der Eintrittsstelle M eingepresst. Die Höhenlage der Eintrittsstelle M definiert den Überdruck in der Gärkammer K1 und macht aufwendige Druckmess- und Steuereinrichtungen überflüssig. Die aufsteigenden Gasblasen verringern die Dichte der Flüssigkeit im Steigrohr 7 gegenüber der umgebenden Flüssigkeit in der Gärkammer K2, wodurch eine nach oben gerichtete Vertikalströmung generiert wird. Das Steigrohr 7 sollte nur geringfügig unter dem Maximalspiegel 11 münden. Die Höhendifferenz zwischen der Eintrittsstelle M der Biogas-Überdruckleitung 9 in das Steigrohr 7 und der Überlauföffnung 5 definiert den Gasdruck in der ersten Gaskammer K1. Sobald sich dieser Gasdruck infolge der biologischen Aktivität aufgebaut hat, strömt das Gas kontinuierlich mit konstantem Druck über die Gasleitung 9 in das Steigrohr 7 der Gärkammer K2. Die aufsteigenden Gasblasen beschleunigen die Vertikalströmung der Flüssigkeit und das entströmt Gas kann nahezu drucklos in die Gasräume der Nachgärkammern K3 und K4 überlaufen und bis hin zu einem außerhalb des Fermenters 2 angeordneten Gasspeicher weiterströmen. Der Effekt der Vertikalströmung wird durch die beheizbaren Steigrohre 6 und 7 noch verstärkt, da diese mit einer Heizeinrichtung 11a und 11 b ausgestattet sind. Im gezeigten Ausführungsbeispiel werden die Heizeinrichtungen 11 a und 11 b von einem Wärmetauscher gebildet, wobei durch die doppelwandig ausgeführten Steigrohre 6 und 7 zwischen den beiden Wandungen ein Heizfluid, vorzugsweise Heizwasser aus einem Heizwasserreservoir 12, zirkulierbar ist. Das Heizwasserreservoir 12 speist somit beide Steigrohre 6 und 7, wobei durch diesen Wärmeeintrag die Strömung begünstigt und andererseits der Wärmeübergang an den umströmten Kontaktflächen erleichtert wird. Das Steigrohr 6 der ersten Gaskammer K1 weist eine Pressluftzufuhr 13 auf, wobei die Lufteinpressung in das Steigrohr 6 der ersten Gärkammer K1 den Anfangspunkt einer Zwangsführung der Entschwefelungsluft durch die Gastürme aller vier Gärkammern K1 bis K4 darstellt. Mit Hilfe des geringfügigen Sauerstoffanteils wird eine mikrobielle Oxidation des H₂S-Schwefels an den Turmoberflächen ermöglicht und durch die Vermeidung von Kurzschlussströmen von Biogas oder Luft ein Rohrentschwefelungsgrad sichergestellt. Entlang des Fließweges durch die Gastürme der vier Kammern K1 - K4 stehen ausreichend Reaktionsflächen zur H₂S-Oxidation zur Verfügung und der ausgefällte Elementarschwefel gelangt zurück in die Biogülle. Die Druckluftmenge ist zwar wesentlich geringer als die produzierte Druckgasmenge aus der Gärkammer K1, die Einleitung der Druckluft kann jedoch wesentlich tiefer, nämlich an der unteren Mündung des Steigrohres 6 erfolgen. Damit erreicht der Thermo-Gas-Lift in der Gärkammer K1 eine ähnliche Förderleistung wie jene in der Gärkammer K2.

Gemäß einem Ausführungsbeispiel der Erfindung kann vorgesehen sein, dass die Gasleitung 9 ein - vorzugsweise zeitweise öffenbares - Überströmventil umfasst, mit dem der Gasdruck der ersten und zweiten Gärkammer K1, K2 ausgleichbar ist. Dies kann beispielsweise durch eine von der Gasleitung 9 abzweigende Bypassleitung erfolgen, wobei das Gas direkt in die zweite Gärkammer K2 einbringbar ist. Durch den eintretenden Gasdruck wird der Flüssigkeitsspiegel der Gärkammer K2 nach unten gepresst, woraufhin ein entsprechendes Flüssigkeitsvolumen ausgehend von der zweiten Gärkammer K2 durch den Spalt 8 der Tauchwand W1 hindurch in die erste Gärkammer K1 gedrückt wird. Dadurch wird insbesondere die Bodenschlammschicht der beiden Gärkammern K1, K2 in eine erhöhte Strömungsgeschwindigkeit versetzt, sodass das bodennahe Substrat zumindest in zeitlichen Intervallen mobilisiert wird und sich Feststoffe nicht dauerhaft am Boden absetzen können.

Die vorgeschlagene Biogasanlage mit ihrem 4-Kammer-Schema bewirkt eine so genannte Pfropfenströmungscharakteristik, d.h. im Gegensatz zu einem vollkommen durchmischten Reaktor wird eine Mindestaufenthaltszeit des Substrates sichergestellt und hydraulische Kurzschlüsse vermieden, wodurch ein vollständigerer Abbau erreicht wird (höhere Biogasausbeute, höhere Qualität der Biogülle hinsichtlich hygienischer Parameter und Geruchsstoffe). Durch die konzentrische Anordnung der vier Gärkammern (Gärkammern K1 und K2 mit größtem Gasumsatz im Kern, Nachgärkammern K3 und K4 an der Peripherie) und ein optimales Volumen/Oberflächenverhältnis (>1) werden Wärmeverluste minimiert und erlauben Temperaturgradiente zwischen Kern und Peripherie. Im Weiteren wird durch die hydraulische Entkopplung von Kern und Peripherie (Überlauf von Gülle und Gas ohne Rücklaufführung) eine hohe Volümsflexibilität erreicht. Das beschriebene Rührsystem fördert Impfschlamm von Gärkammer K2 in Gärkammer K1 und daher kann der Kern unabhängig betrieben werden, d.h. die Gärkammern K3 und K4 können sowohl als Reaktionsvolumen als auch als gasdichtes Endlager genutzt und entleert werden.

Die vorliegende Erfindung beschränkt sich nicht auf das gezeigte Ausführungsbeispiel, sondern umfasst bzw. erstreckt sich auf alle Varianten und technischen Äquivalente, die in die Reichweite der nachfolgenden Ansprüche fallen können. Auch sind die in der Beschreibung gewählten Lageangaben, wie z.B. oben, unten, usw. auf die übliche Einbaulage des Fermenters bzw. auf die unmittelbar beschriebene sowie dargestellte Figur bezogen und sind bei einer Lageänderung sinngemäß auf die neue Lage zu übertragen. Es können auch passive Mischeinrichtungen wie beispielsweise ein Lochgitter vorgesehen werden, das in den Gärkammern K1 und K2 im Schwimmdeckenbereich des Gärmediums angeordnet ist. Bei einem vom Überströmventil ausgelösten Druckausgleich zwischen den Gärkammern K1 und K2 schlägt das Gärmedium durch das Lochgitter durch und verhindert dadurch ein Verfestigen der Schwimmdecke. '

## Patentansprüche

1. Biogasanlage (1) mit einem Fermenter (2), der eine erste Gärkammer (K1) und zumindest eine zweite Gärkammer (K2) zum Vergären des Gärmediums aufweist, wobei in der ersten Gärkammer (K1) gebildetes Biogas in ein in der zweiten Gärkammer (K2) angeordnetes Steigrohr (7) einbringbar ist, **dadurch gekennzeichnet, dass** eine Gasleitung (9) von der ersten Gärkammer (K1) zur zweiten Gärkammer (K2) führt und an einer Eintrittsstelle (M) in das in der zweiten Gärkammer (K2) angeordnete Steigrohr (7) mündet, sodass in der ersten Gärkammer (K1) gebildetes Biogas in das Steigrohr (7) einbringbar ist, wobei das Steigrohr (7) eine Einlassöffnung (E) und eine Auslassöffnung (A) zum Durchtritt des Gärmediums aufweist.

2. Biogasanlage nach Anspruch 1, **dadurch gekennzeichnet, dass** das Steigrohr (7) in der Gärkammer (K2) im Wesentlichen vertikal und vorzugsweise mittig angeordnet ist.

3. Biogasanlage nach Anspruch 1 oder 2, **dadurch gekennzeichnet, dass** die Eintrittsstelle (M) etwa im unteren Bereich, vorzugsweise in der untersten Hälfte, des obersten Drittels des Steigrohres (7) angeordnet ist.

4. Biogasanlage nach einem der Ansprüche 1 bis 3, **dadurch gekennzeichnet, dass** das Steigrohr (7) eine Heizeinrichtung (11b), vorzugsweise einen Wärmetauscher, aufweist.

5. Biogasanlage nach einem der Ansprüche 1 bis 4, **dadurch gekennzeichnet, dass** das Steigrohr (7) zumindest bereichsweise doppelwandig ausgeführt ist, wobei zwischen den beiden Wandungen ein Heizfluid, vorzugsweise Heizwasser, zirkulierbar ist.

6. Biogasanlage nach einem der Ansprüche 1 bis 5, **dadurch gekennzeichnet, dass** die Gasleitung (9) bis auf einen Einlass und einen Auslass geschlossen ist.

7. Biogasanlage nach einem der Ansprüche 1 bis 6, **dadurch gekennzeichnet, dass** die erste Gärkammer (K1) wenigstens im befüllten Zustand - bis auf die Gasleitung (9) - gasdicht ausgebildet ist.

8. Biogasanlage nach einem der Ansprüche 1 bis 7, **dadurch gekennzeichnet, dass** die Gasleitung (9) ein, vorzugsweise zeitweise öffenbares, Überströmventil umfasst, mit dem der Gasdruck zwischen der ersten Gärkammer (K1) und der zweiten Gärkammer (K2) ausgleichbar ist.

9. Biogasanlage nach einem der Ansprüche 1 bis 8, **dadurch gekennzeichnet, dass** die erste Gärkammer (K1) und die zweite Gärkammer (K2) durch eine Wand (W1), vorzugsweise eine Tauchwand, voneinander getrennt sind, sodass die erste Gärkammer (K1) - bis auf die Gasleitung (9) -oben gasdicht ausgebildet ist und unten den Durchtritt des Gärmediums von einer Gärkammer (K1, K2) in die andere erlaubt.

10. Biogasanlage nach Anspruch 9, **dadurch gekennzeichnet, dass** die Tauchwand (W1) im untersten Bereich, vorzugsweise im untersten Viertel, eine Öffnung (8), vorzugsweise einen über die Breite der Tauchwand (W1) erstreckenden Spalt, aufweist, die zum Durchtritt des Gärmediums von einer Gärkammer (K1, K2) in die andere vorgesehen ist.

11. Biogasanlage nach einem der Ansprüche 1 bis 10, **dadurch gekennzeichnet, dass** in der ersten Gärkammer (K1) ebenfalls ein Steigrohr (6) angeordnet ist.

12. Biogasanlage nach Anspruch 11, **dadurch gekennzeichnet, dass** in das Steigrohr (6) der ersten Gärkammer (K1) Pressluft einbringbar ist.

13. Biogasanlage nach einem der Ansprüche 1 bis 12, **dadurch gekennzeichnet, dass** die erste und die zweite Gärkammer (K1, K2) gemeinsam im Wesentlichen eine Rundform bilden.

## Claims

1. A biogas plant (1) having a fermenter (2), which has a first fermentation chamber (K1) and at least a second fermentation chamber (K2) for fermenting the fermentation medium, wherein biogas that is produced in the first fermentation chamber (K1) may be introduced into an ascending tube (7) arranged in the second fermentation chamber (K2), **characterized in that** a gas pipe (9) extends from the first fermentation chamber (K1) to the second fermentation chamber (K2) and discharges at an entry port (M) into the ascending tube (7) arranged in the second fermentation chamber (K2) so that biogas that is produced in the first fermentation chamber (K1) may be introduced into the ascending tube (7), wherein the ascending tube (7) has an inlet opening (E) and an outlet opening (A) for the passage of the fermentation medium.

2. A biogas plant according to claim 1, **characterized in that** the ascending tube (7) is arranged in the fermentation chamber (K2) substantially vertically and preferably centrally.

3. A biogas plant according to claim 1 or 2, **characterized in that** the entry port (M) is arranged approximately in the lower area, preferably in the bottom half, of the top third of the ascending tube (7).

4. A biogas plant according to any of claims 1 to 3, **characterized in that** the ascending tube (7) has a heating device (11b), preferably a heat exchanger.

5. A biogas plant according to any of claims 1 to 4 **characterized in that** the ascending tube (7) is formed with double-walls at least in some sections, wherein a heating fluid, preferably heating water, may be circulated between the two walls.

6. A biogas plant according to any of claims 1 to 5, **characterized in that** the gas pipe (9) is closed except for one inlet and one outlet.

7. A biogas plant according to any of claims 1 to 6, **characterized in that** the first fermentation chamber (K1) is formed gas-tight at least in the filled state - except for the gas pipe (9).

8. A biogas plant according to any of claims 1 to 7, **characterized in that** the gas pipe (9) comprises a waste gate, which may preferably be opened temporarily, by means of which the gas pressure may be balanced between the first fermentation chamber (K1) and the second fermentation chamber (K2).

9. A biogas plant according to any of claims 1 to 8, **characterized in that** the first fermentation chamber (K1) and the second fermentation chamber (K2) are separated from each other by a wall (W1), preferably a baffle, so that the first fermentation chamber (k1) is formed gas-tight at the top - except for the gas pipe (9) - and allows for the passage of the fermentation medium from one fermentation chamber (K1, K2) into the other one.

10. A biogas plant according to claim 9 **characterized in that** the baffle (W1) has in the bottom area, preferably in the lowest quarter, an opening (8), preferably a gap extending across the width of the baffle (W1), which is provided for the passage of the fermentation medium from one fermentation chamber (K1, K2) into the other one.

11. A biogas plant according to any of claims 1 to 10, **characterized in that** there is also arranged an ascending tube (6) in the first fermentation chamber (K1).

12. A biogas plant according to claim 11, **characterized in that** compressed air may be introduced into the ascending tube (6) of the first fermentation chamber (K1).

13. A biogas plant according to any of claims 1 to 12, **characterized in that** the first and the second fermentation chamber (K1, K2) together form substantially a round shape.

## Revendications

1. Installation de biogaz (1) comprenant un fermenteur (2), qui comprend une première chambre de fermentation (K1) et au moins une seconde chambre de fermentation (K2) pour faire fermenter le milieu de fermentation, dans laquelle le biogaz formé dans la première chambre de fermentation est susceptible d'être introduit dans un tube montant (7) agencé dans la seconde chambre de fermentation (K2), **caractérisée en ce qu'**un conduit de gaz (9) mène depuis la première chambre de fermentation (K1) vers la seconde chambre de fermentation (K2) et débouche à un emplacement d'entrée (M) dans le tube montant (7) agencé dans la seconde chambre de fermentation (K2), de sorte que le biogaz formé dans la première chambre de fermentation (K) peut être introduit dans le tube montant (7), et dans laquelle le tube montant (7) comporte une ouverture d'entrée (E) et une ouverture de sortie (A) pour la traversée du milieu de fermentation.

2. Installation de biogaz selon la revendication 1, **caractérisée en ce que** le tube montant (7) est agencé sensiblement verticalement et de préférence au milieu dans la chambre de fermentation (K2).

3. Installation de biogaz selon la revendication 1 ou 2, **caractérisée en ce que** l'emplacement d'entrée (M) est agencé environ dans la zone inférieure, de préférence dans la moitié la plus inférieure, du plus haut tiers du tube montant (7).

4. Installation de biogaz selon l'une des revendications 1 à 3, **caractérisée en ce que** le tube montant (7) comprend un dispositif de chauffage (11b), de préférence un échangeur de chaleur.

5. Installation de biogaz selon l'une des revendications 1 à 4, **caractérisée en ce que** le tube montant (7) est réalisé au moins localement à double paroi, et un fluide chauffant, de préférence de l'eau de chauffage, est amené à circuler entre les deux parois.

6. Installation de biogaz selon l'une des revendications 1 à 5, **caractérisée en ce que** le conduit de gaz (9) est fermé, à l'exception d'une entrée et d'une sortie.

7. Installation de biogaz selon l'une des revendications 1 à 6, **caractérisée en ce que** la première chambre de fermentation (K1) est réalisée étanche aux gaz, au moins dans l'état rempli, à l'exception du conduit de gaz (9).

8. Installation de biogaz selon l'une des revendications 1 à 7, **caractérisée en ce que** le conduit de gaz (9) inclut une vanne de déversement, de préférence susceptible d'être ouverte temporairement, avec laquelle la pression du gaz peut être égalisée entre la première chambre de fermentation (K1) et la seconde chambre de fermentation (K2).

9. Installation de biogaz selon l'une des revendications 1 à 8, **caractérisée en ce que** la première chambre de fermentation (K1) et la seconde chambre de fermentation (K2) sont séparées l'une de l'autre par une cloison (W1), de préférence une cloison plongeante, de sorte que la première chambre de fermentation (K1) est réalisée étanche aux gaz en partie haute, à l'exception du conduit de gaz (9), et permet en bas la traversée du milieu de fermentation depuis une chambre de fermentation (K1, K2) vers l'autre.

10. Installation de biogaz selon la revendication 9, **caractérisée en ce que** la cloison plongeante (W1) comporte dans la région la plus inférieure, de préférence dans le quart le plus inférieur, une ouverture (8), de préférence une fente qui s'étend sur la largeur de la cloison plongeante (W1), qui est prévue pour la traversée du milieu de fermentation depuis une chambre de fermentation (K1, K2) vers l'autre.

11. Installation de biogaz selon l'une des revendications 1 à 10, **caractérisée en ce qu'**un tube montant (6) est également agencé dans la première chambre de fermentation (K1).

12. Installation de biogaz selon la revendication 11, **caractérisée en ce que** de l'air comprimé est susceptible d'être introduit dans le tube montant (6) de la première chambre de fermentation (K1).

13. Installation de biogaz selon l'une des revendications 1 à 12, **caractérisée en ce que** la première et la seconde chambre de fermentation (K1, K2) forment conjointement sensiblement une forme ronde.
